(19) Europäisches Patentamt
European Patent Office
Office européen des brevets

(11) Veröffentlichungsnummer: **0 088 252**
**B1**

(12) # EUROPÄISCHE PATENTSCHRIFT

(45) Veröffentlichungstag der Patentschrift: **09.04.86**

(51) Int. Cl.⁴: **C 07 D 209/28**

(21) Anmeldenummer: **83101353.7**

(22) Anmeldetag: **12.02.83**

(54) **Verfahren zur Herstellung von 1-(4-Chlorbenzoyl)-5-methoxy-2-methyl-3-indolacetoxyessigsäure.**

(30) Priorität: **26.02.82 DE 3206886**

(43) Veröffentlichungstag der Anmeldung:
**14.09.83 Patentblatt 83/37**

(45) Bekanntmachung des Hinweises auf die Patenterteilung:
**09.04.86 Patentblatt 86/15**

(84) Benannte Vertragsstaaten:
**AT BE CH DE FR GB IT LI LU NL SE**

(56) Entgegenhaltungen:
**DE - A - 2 740 854**

***CHEMICAL ABSTRACTS*, Band 90, Nr. 10, 5. März 1979, Seite 60, Nr. 87267x, Columbus, Ohio, USA**

**Die Akte enthält technische Angaben, die nach dem Eingang der Anmeldung eingereicht wurden und die nicht in dieser Patentschrift enthalten sind.**

(73) Patentinhaber: **Troponwerke GmbH & Co. KG, Berliner Strasse 156, D-5000 Köln 80 (DE)**

(72) Erfinder: **Boltze, Karl-Heinz, Dr., Ackerstrasse 8, D-5231 Borod (DE)**
Erfinder: **Lehnen, Hans Dieter, Schubertstrasse 11, D-5040 Troisdorf (DE)**

(74) Vertreter: **Jesse, Ralf-Rüdiger, Dr. et al, Bayer AG Konzernverwaltung RP Patentabteilung, D-5090 Leverkusen 1 Bayerwerk (DE)**

**EP 0 088 252 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

Die vorliegende Erfindung betrifft ein neues chemisch eigenartiges und vorteilhaftes Verfahren zur Herstellung der bekannten 1-(4-Chlorbenzoyl)-5-methoXy-2-methyl-3-indolacetoxyessigsäure (Acemetacin entsprechend Formel I).

Für die Herstellung dieser bekannten Verbindung sind bereits eine Reihe von Verfahren bekannt geworden, vgl. z.B. DE-OS 2 234 651, DE-OS 2 257 867 und DE-OS 2 943 125.

Bei den bekannten Verfahren wurde die Carboxygruppe zunächst durch einen Benzylrest geschützt, so daß in einem letzten Reaktionsschritt eine katalytische Hydrierung des Benzylesters gemäß folgendem Reaktionsschema durchgeführt werden mußte.

**Reaktionsschema**

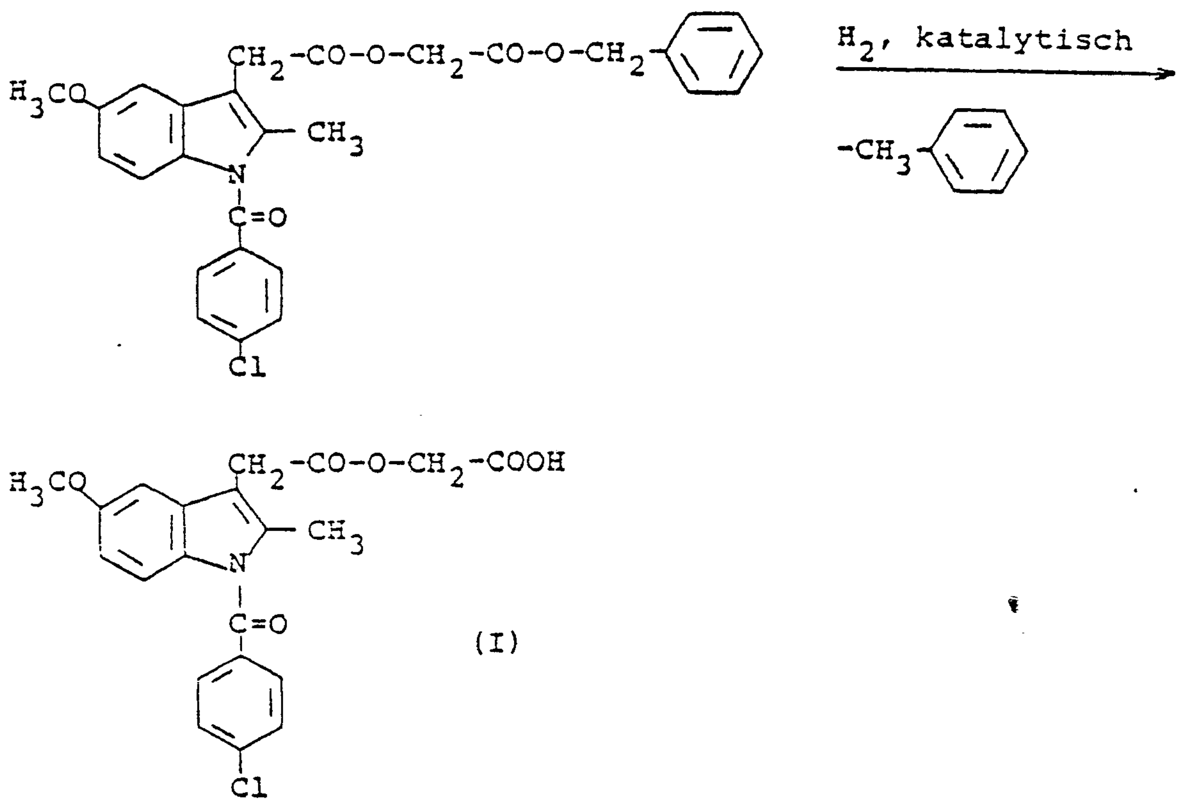


(I)

Bei dieser Abspaltung des Benzylrestes entsteht als Nebenprodukt stets die 1-Benzoyl-5-methoxy-2-methyl-3-indolacetoxyessigsäure, nachstehend Des-Chlor-Verbindung genannt. Diese unerwünschte Verunreinigung, die durch Abspaltung des Chlors aus dem Benzolring des 4-Chlorbenzoylrestes bis zu einer Menge von 0,5 % entsteht, muß in aufwendigen Reinigungsschritten anschließend entfernt werden, was mit Ausbeuteverlusten verbunden ist.

In CA <u>90,</u> 87267x wird die Umsetzung von 1-(p-Chlorbenzoyl-5-methoxy-2-methylindol-3-essigsäure (Indometacin) mit Thionylchlorid im Ethylether/Pyridin beschrieben. Das Umsetzungsprodukt ergibt mit Glykolsäure in trockenem Pyridin 1-(p-Chlorbenzoyl)-5-methoxy-2-methyl-indol-3-acetoxy-essigsäure (Acemetacin).

Es wurde ein Verfahren zur Herstellung von 1-(4-Chlorbenzoyl)-5-methoxy-2-methyl-3-indolacetoxyessigsäure gefunden, das dadurch gekennzeichnet ist, daß man Indolcarbonsäurederivate der allgemeinen Formel

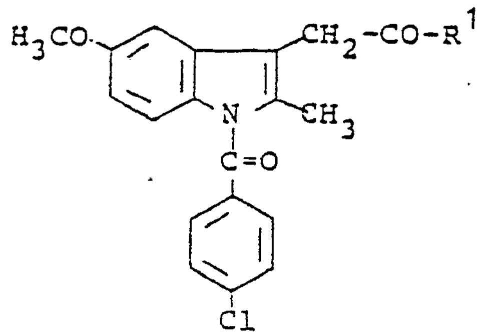

(II),

in welcher
$R^1$ für die Reste

$-O-\overset{O}{\overset{\|}{C}}-OC_2H_5$, $-O-SO_2-CH_3$, 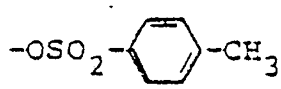

und 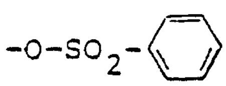 ,steht,

mit Verbindungen der allgemeinen Formel III

$$HO-CH_2-\overset{O}{\overset{\|}{C}}-O-R^2 \qquad (III),$$

in welcher
$R^2$ für Wasserstoff oder Ammonium steht
in Gegenwart von inerten organischen Lösungsmitteln in einem Temperaturbereich von −10°C bis 80°C umsetzt

Inerte organische Lösungsmittel für das erfindungsgemäße Verfahren können Ether wie Diethylether, Diisopropylether, Dioxan, Tetrahydrofuran und 1,2-Dimethoxyethan, chlorierte Kohlenwasserstoffe wie Methylenchlorid, Chloroform und Dichlorethan, substituierte Amide wie Dimethylformamid und N-Methylpyrrolidon, Aromaten wie Toluol und Mylol, Ketone wie Aceton und Methylethylketon (Butanon-2) sein. Das erfindungsgemäße Verfahren wird in einem Temperaturbereich von −10°C bis 80°C, bevorzugt bei −10°C bis 50°C, besonders bevorzugt bei −5°C bis 20°C, durchgeführt.

Verwendet man als Vertreter der allgemeinen Formel II die Indolcarbonsäurederivate, bei denen $R^1$ die obengenannten Substituenten bedeutet und Verbindungen der allgemeinen Formel III als Ausgangsstoffe, so kann der Reaktionsablauf durch das folgende Formelschema wiedergegeben werden:

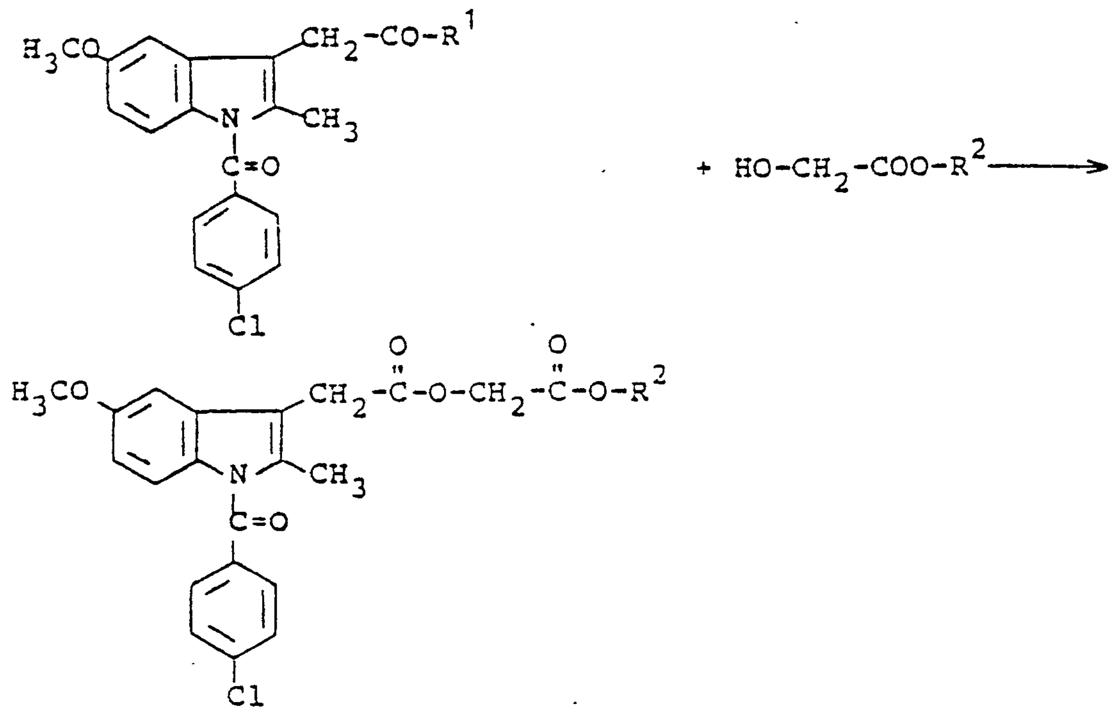

$R^1$ besitzt die oben angegebene Bedeutung, bevorzugt

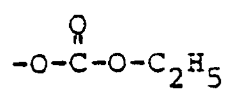

$R^2$ besitzt die oben angegebene Bedeutung, vorzugsweise Ammoniumkation.

Die entstandenen Ammoniumverbindungen werden anschließend durch Behandlung mit Säuren in einfacher Weise in das Endprodukt I überführt.

Es war überraschend, daß nach diesen Verfahren die 1-(4-Chlorbenzoyl)-5-methoxy-2-methyl-3-indolacetoxyessigsäure in so reiner Form, d.h. frei von der störenden Des-Chlor-Verbindung, und in Ausbeuten von 60-70 % der Theorie entsteht.

Die als Ausgangsstoffe verwendeten Verbindungen der Formel II und III sind bekannt oder werden nach bekannten Verfahren hergestellt.

Die nach den erfindungsgemäßen Verfahren hergestellte Endverbindung I ist ein wertvoller pharmazeutischer Wirkstoff mit antiphlogistischer Wirkung, vgl. z.B. DE-PS 2 234 651.

Im vorliegenden Verfahren wird die Indolcarbonsäure II ($R^1$ = OH) durch Anhydridbildung mit vorzugsweise Ethylcarbonat funktionalisiert. Hierzu setzt man ein Ammoniumsalz von II, bevorzugt das N-Methylmorpholiniumsalz, mit Chlorameisemsäureethylester um (vgl. Reaktionsschema: Synthese von I). Das entstandene hochaktive, bisher noch nicht bekannte gemischte Anhydrid wird isoliert und rein dargestellt.

Seine Reaktion mit Glykolsäure III ($R^2$= H) Führt nicht zu dem gewünschten Reaktionsprodukt I. Erst in der Glykolatform mit Aminen, bevorzugt mit dem Diisopropylamin, gelingt die Umsetzung der OH-Gruppe in α-Stellung der Glykolsäure im Sinn der Abspaltung des gemischten Anhydrids unter Bildung von Diisopropylammonium 1-(4-Chlorbenzoyl)-5-methoxy-2-methyl-3-indolacetoxyacetat, aus dem durch Behandlung mit Säuren die Endverbindung I in Freiheit gesetzt wird.

**Reaktionsschema:**

Synthese von I (gemischtes-Anhydrid-Verfahren) (Stufen a-d)

a)

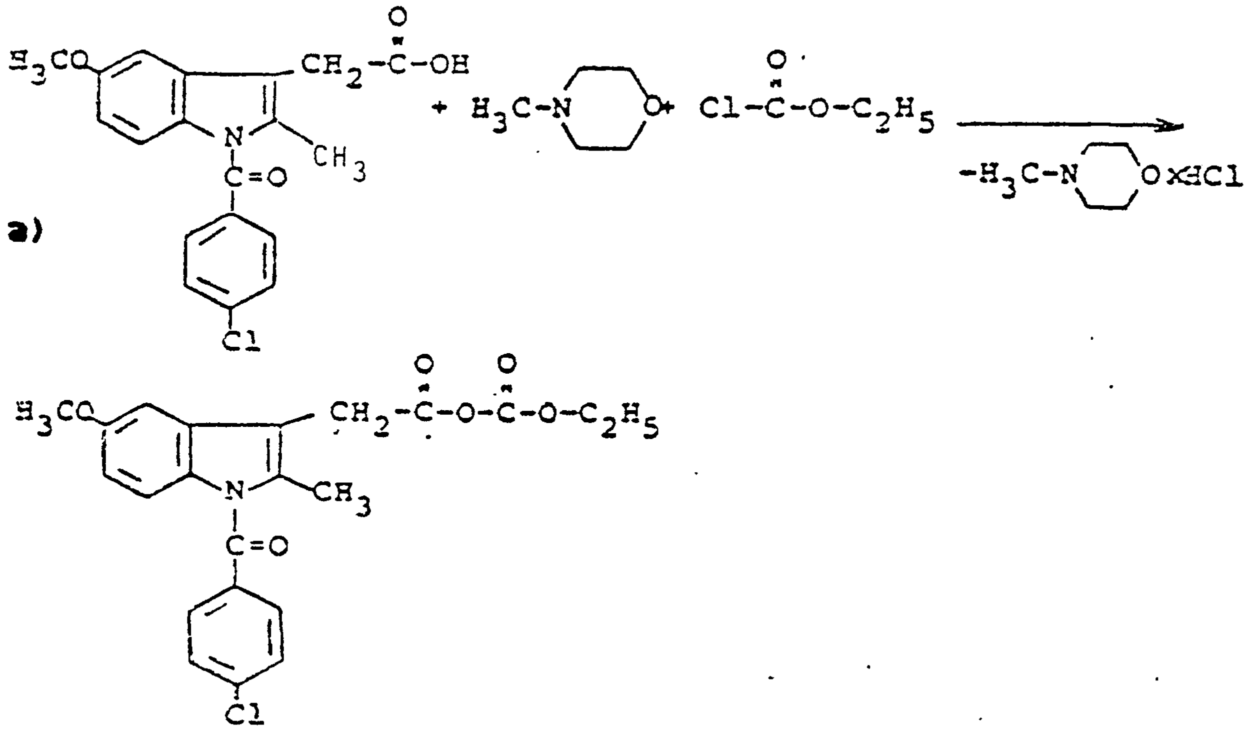

b) 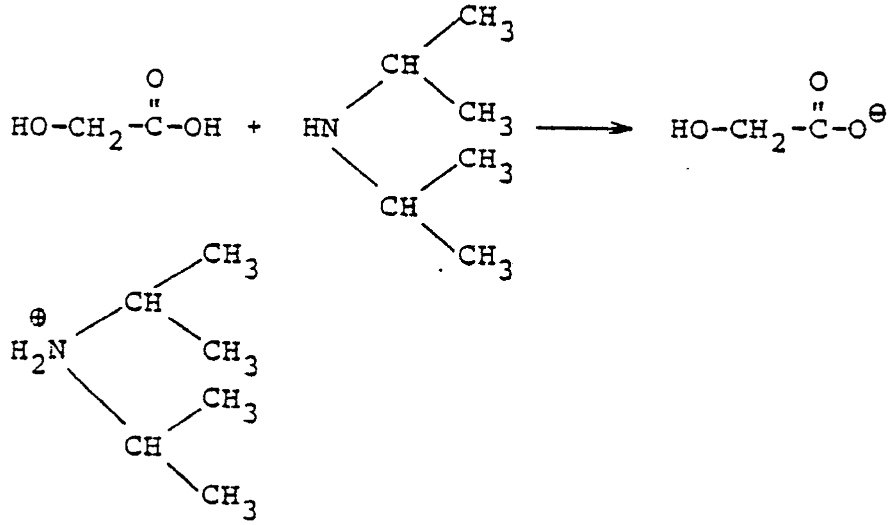

c) a + b 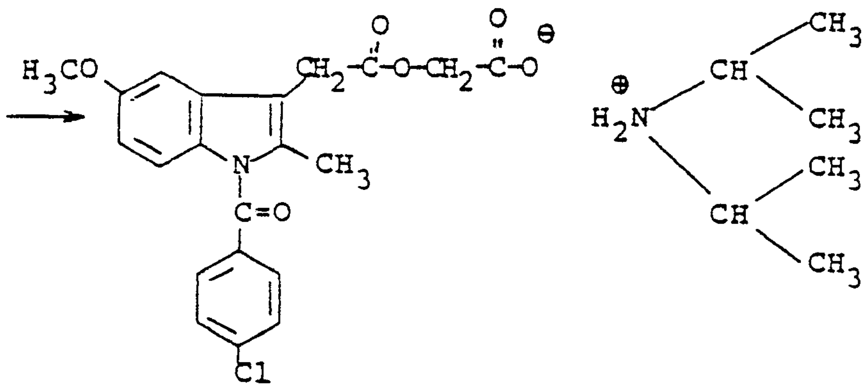

d) + HCl

Trocknung

I

In weiterer Ausbildung des Verfahrens wurden gemischte Anhydride mit Sulfonsäuren (II $R^1$-O-$SO_2CH^3$,

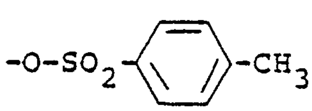 und 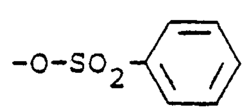 )

eingesetzt, indem

anstelle von Chlorameisensäureethylester Methansulfonsäurechlorid, p-Toluolsulfonsäurechlorid und Benzolsulfonsäurechlorid für die Herstellung der gemischten Anhydride verwendet wurden.

Beispielhaft für diese Varianten wird die Herstellung von I über das 1-(4-Chlorbenzoyl)-5-methoxy-2-methyl-indol-3-essigsäureanhydrid mit Benzolsulfonat (vgl. Reaktionsschema) beschrieben.

1. Reaktionsschema: Synthese von I (Gemischte-Anhydrid-Verfahren (Fortsetzung)
(Stufen: a-d)

a)

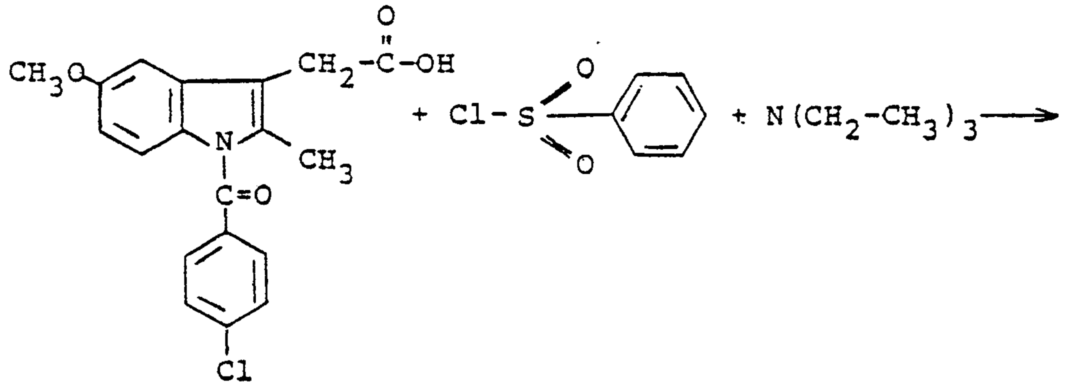

b)

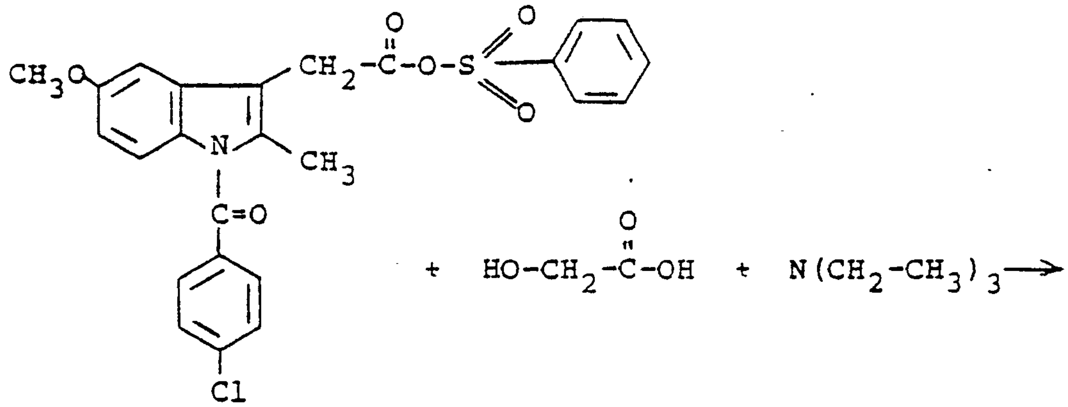

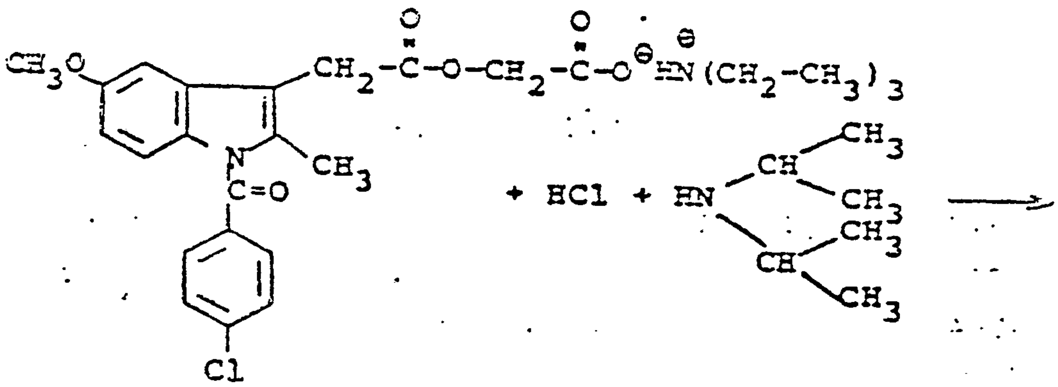

c)

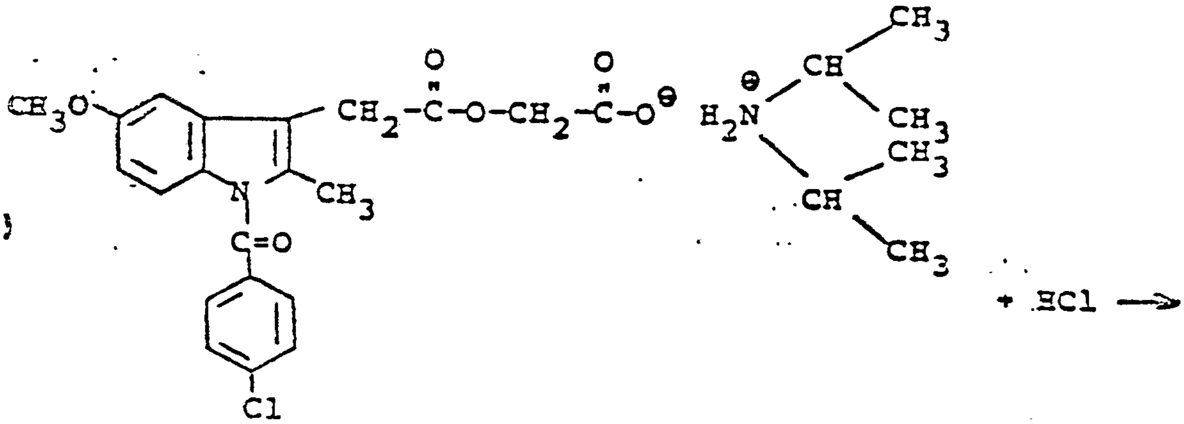

d).

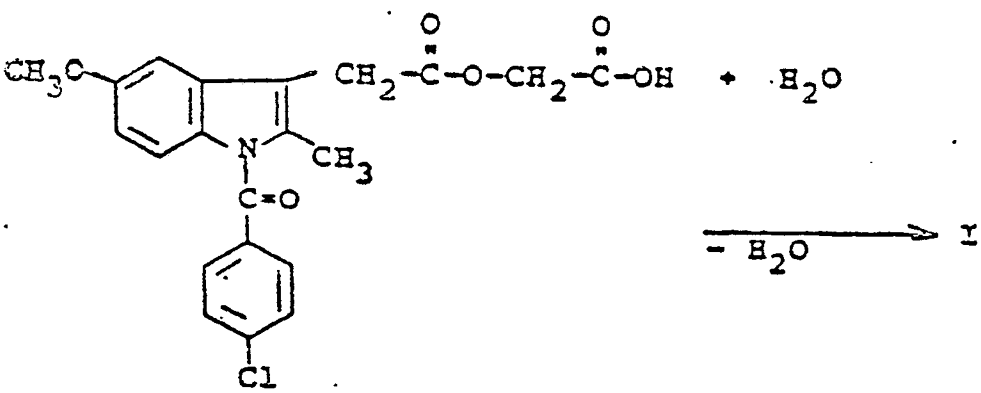

**Beispiel 1**

a) gemischtes Anhydrid

$$(II,\ R^1-O-\overset{O}{\overset{\|}{C}}-O-C_2H_5)$$

10,8 g II ($R^1$ OH) (0,03 Mol) und 3,3 ml N-Methylmorpholin (0,03 Mol) werden in 70 ml abs. Tetrahydrofuran (THF) gelöst. Zu dieser Lösung tropft man unter Rühren und Feuchtigkeitsabschluß bei ca. —10°C die Lösung von 3,0 ml Chlorameisensäureethylester (0,03 Mol) in 5 ml THF und läßt 15 Minuten bei —8°C nachreagieren.

Das ausgefallene N-Methylmorpholinhydrochlorid wird durch Filtration entfernt und das Filtrat im Rotationsverdampfer bei Wasserstrahlvakuum und 20°C eingedampft. Der sirupöse Rückstand wird mit 50 ml abs. Aceton aufgenommen und unter Rühren auf -15°C abgekühlt. Hierbei entsteht ein Kristallisat, getrocknet über $P_2O_5$.

**Ausbeute:**

1,7 g II-Anhydrid (II-O-II) (symmetrisch);

Das Filtrat wird wiederum unter Vakuum bei 20°C eingedampft und der Rückstand in 50 ml abs. Ether gelöst. Nach Stehen bei -15°C bilden sich Kristalle, Trocknung über $P_2O_5$.

**Ausbeute:**

9 g II,

$$R^1-O-\overset{O}{\overset{\|}{C}}-O-C_2H_5,$$

69,7 % d. Th., Mikro F: 74-75°C (Zers.).

b) Diisopropylammoniumglykolat

Zu 392 ml (4,0 Mol) Glykolsäure III ($r^2$ H) werden unter Rühren, $CO_2$-Abschluß und Kühlung 650 ml (4,532 Mol) Diisopropylamin bei einer Temperatur von ca. 60°C eingetropft. Das Gemisch erhitzt man 3 h unter Rückfluß (Badtemperatur 120°C). Dem auf Raumtemperatur abgekühlten Reaktionsgemisch setzt man unter Rühren 1 1 Aceton zu und kühlt ab auf +5°C. Nach Adimpfen erfolgt Kristallisation, die unter Rühren (1 h) vervollständigt wird. Die abfiltrierte Substanz wird zweimal mit je 500 ml Aceton gewaschen und im Exsikkator bei 40°C über $P_2O_5$ getrocknet.

**Ausbeute:**

625 g Diisopropylammoniumglykolat 88,15 % d.Th., Mettler Fp 61: 79,9°C, farblose, kristalline geruchsfreie Substanz.

c) Diisopropylammonium 1-(4-Chlorbenzoyl)-5-methoxy-2-methyl-3-indolacetoxyacetat

140 ml Chlorameisensäureethylester (1,4 Mol) wer-den unter Rühren in 1 1 Aceton gelöst und bei einer Temperatur von 15-18°C innerhalb von 30 Minuten, mit einer Lösung von 504 g II ($R_1$ OH) (1,408 Mol) und 154 ml N Methylmorpholin in 1 l Aceton versetzt. Man läßt 3 Minuten bei einer Temperatur von +15°C nachreagieren und filtriert den entstandenen Niederschlag ab. Der Filterrückstand wird zweimal mit je 125 ml Aceton gewaschen.

Die vereinigten gelben Filtrate tropft man anschliessend unter Rühren bei +25 - 27°°C innerhalb von 2,5 h zu einer Lösung von 413 g Diisopropylammoniumglykolat (2,33 Mol) in 1 l $CH_2Cl_2$ und läßt 1 h reagieren

Die Reaktionslösung wird im Rotationsverdampfer unter Wasserstrahlvakuum bei 30-40°C vom Lösungsmittel befreit und der sirupöse Rückstand mit 3 1 Ether unter Rühren versetzt. Nach kurzer Zeit erfolgt Kristallisation. Die farblose kristalline Substanz wird abfiltriert und zweimal mit je 1,5 1 Ether gewaschen. Zur Reinigung löst man die Substanz unter Rühren und Rückfluß in 1,5 1 Butanon-(2), läßt auf Raumtemperatur abkühlen und leitet durch Animpfen die Kristallisation ein, die nach Zufügen von 3 l Diisopropylether vervollständigt wird.

d) Überführung in 1-(4-Chlorbenzoyl)-5-methoxy-2-methyl-3-indolacetoxyessigsäure (I)

Das so gewonnene Ammoniumsalz (608 g) löst man unter Rühren in einem Gemisch von 2,42 l Aceton und 0,769 l $H_2O$.

Anschließend gibt man 400 ml 1 n HCl zu dieser Lösung sung und setzt Impfkristalle zu. Innerhalb 1 Stunde

tropft man weiterhin bei 20°C 1 l 1 n HCl zu (pH: 3) ständig ist. Die farblose kristalline Substanz wird abgesaugt, mit $H_2O$ gewaschen und bei 40°C im Wasserstrahlvakuum getrocknet.

**Ausbeute:**

410 g I-Monohydrat = 67,1 % d. Th.
In einem Trocknungsprozeß bei 40°C (1 h) findet die vollständige Entwässerung unter Bildung von I statt: gelbliche Kristalle, die bei 151-152°C schmolzen.

**Beispiel 2**

a) gemischtes Anhydrid

(II, 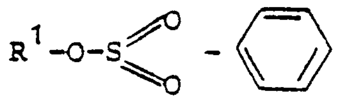 )

b) Umsetzung mit Triethylammoniumglykolat
c) Umwandlung in das Diisopropylammoniumsalz von I (Reaktionsfolge a-c ohne Isolierung der Zwischenprodukte)

Zu einer Lösung von 2,9 g Benzolsulfonsäurechlorid (0,015 Mol) in 40 ml abs. Methylenchlorid ($CH_2Cl_2$) gibt man unter Rühren und Feuchtigkeitsausschluß bei 0°C tropfenweise eine Lösung von 5,4 g II ($R^1$ OH) (0,015 Mol) und 2,07 ml Triethylamin (0,015 Mol) in 20 ml $CH_2Cl_2$. Die Reaktion läuft in 1 1/2 h bei Raumtemperatur ab. Anschließend wird innerhalb 15 Minuten unter Rühren eine Lösung von 2,3 g Glykolsäure (0,03 Mol) und 4,14 ml Triethylamin (0,03 Mol) in 15 ml $CH_2Cl_2$ zugetropft und danach noch 19 h bei 28°C weitergerührt. Zur Aufarbeitung wird die Reaktionslösung mit 35 ml 1 n HCl angesäuert und anschließend zweimal mit je 50 ml $H_2O$ gewaschen. Nach Trocknen der Methylenchloridphase mit $Na_2SO_4$ wird die Lösung mit 2,1 ml Diisopropylamin (0,015) versetzt und im Rotationsverdampfer $CH_2Cl_2$ abdestilliert. Der Rückstand wird in 30 ml Aceton gelöst und nach Animpfen und Rühren (1 h bei Raumtemperatur) wird die kristalline farblose Substanz abgesaugt und bei 40°C im Exsikkator getrocknet.

**Ausbeute:**

3,6 g Diisopropylammonium 1(4-Chlorben-zoyl-5-methoxy-2-methyl-3-indolacetoxyacetat
d) Überführung in I-Monohydrat und Entfernung des Hydratwassers

3,6 g des Ammoniumsalzes werden unter Rühren in 14 ml Aceton und 4,6 ml $H_2O$ gelöst. Anschließend gibt man 2,6 ml 1 n HCl zu dieser Lösung und impft an. Innerhalb 1 h tropft man 4,4 ml 1 n HCl zu und rührt 1 h nach, bis die Kristallisation vollständig ist. Die farblose kristalline Substanz wird abgesaugt, mit $H_2O$ gut gewaschen und im Exsikkator bei 40°C im Wasserstrahlvakuum getrocknet.

**Ausbeute:**

2,3 g I-Monohydrat = 35,6 % d. Th. Nach Trocknung bei 90° unter Wasserstrahlvakuum (1 h) erhält man gelbliche Kristalle, die bei 151-152°C schmelzen.
In analoger Verfahrensweise werden die gemischten Anhydride

(II, 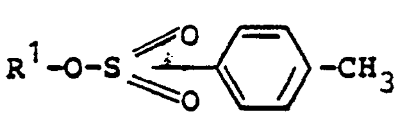 und 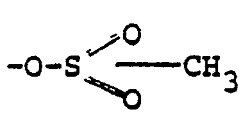) einge-

setzt und die Endverbindung I in 20-53 %iger Ausbeute erhalten.

**Patentansprüche**

1. Verfahren zur Herstellung von 1-(4-Chlorbenzoyl)-5-methoxy-2-methyl-3-indolacetoxyessigsäure (I), dadurch gekennzeichnet, daß man Indolcarbonsäurederivate der allgemeinen Formel

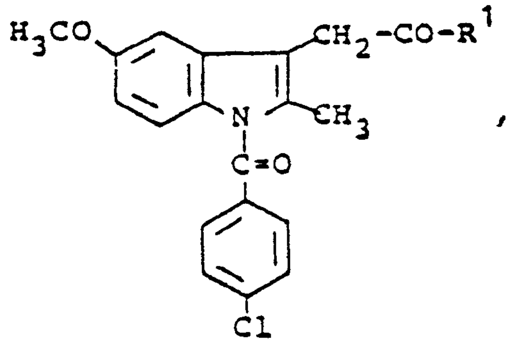
,

in welcher $R^1$ für die Reste

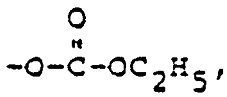
,

$-O-SO_2-CH_3$,

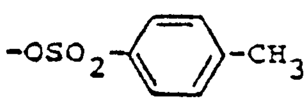

und

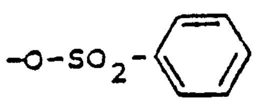

steht,
mit Verbindungen der allgemeinen Formel

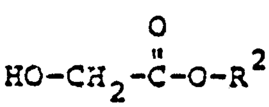

in welcher
$R^2$ für Wasserstoff oder Ammonium steht
in Gegenwart von inerten organischen Lösungsmitteln in einem Temperaturbereich Von −10°C bis 80°C umsetzt

2. Verfahren nach Anspruch 1, dadurch gekennzeichnet daß die Umsetzung im Temperaturbereich von −10°C bis +50°C vorgenonmen wird.

3. Verfahren nach Anspruch 1 oder 2, dadurch gekennzeichnet, daß als inerte organische Lösungsmittel Ether, chlorierte- Kohlenwasserstoffe, substituierte Amide, Aromaten und/oder Ketone eingesetzt werden.

4. Verfahren nach Ansprüchen 1 bis 3, dadurch gekennzeichnet, daß man als Indolcarbonsäurederivat eine durch Anhydridbildung mit Ethylcarbonat funktionalisierte Indolcarbonsäure einsetzt.

5. Verfahren nach Ansprüchen 1 bis 4, dadurch gekennzeichnet, daß man ein Ammoniumsalz der Glykolsäure einsetzt.

## Claims

1. Process for the preparation of 1-(4-chlorobenzoyl)-5-methoxy-2-methyl-3-indoleacetoxyacetic acid (I), characterised in that indolecarboxylic acid derivatives of the general formula

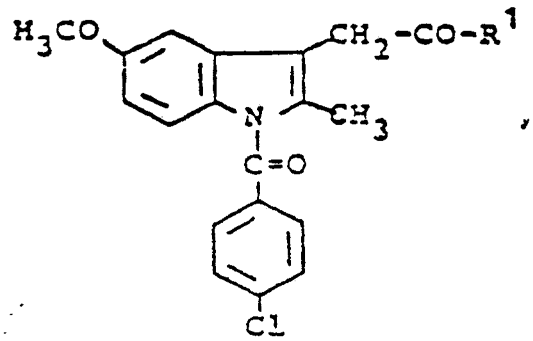

in which
$R^1$ represents the radicals

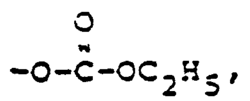

$-O-SO_2-CH_3$,

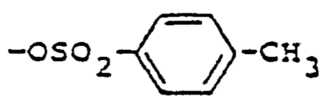

and

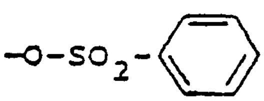

are reacted with compounds of the general formula

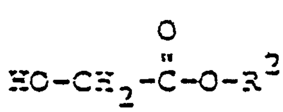

in which
$R^2$ represents hydrogen or ammonium, in the presence of inert organic solvents in a temperature range of −10°C to 80°C

2. Process according to Claim 1, characterised in that the reaction is carried out in a temperature range of −10°C to +50°C.

3. Process according to Claim 1 or 2, characterised in that ethers, chlorinated hydrocarbons, substituted amides, aromatics and/or ketones are used as the inert organic solvents.

4. Process according to Claims 1 to 3, characterised in that an indolecarboxylic acid functionalised by forming the anhydride With ethyl carbonate is used as the indolecarboxylic acid derivative.

5. Process according to Claims 1 to 4, characterised in that an ammonium salt of glycolic acid is used.

**Revendications**

1. Procédé de production d'acide 1-(4-chloro-benzoyl)-5-méthoxy-2-méthyl-3-indolacétoxyacétique (I), caractérisé en ce qu'on fait réagir des dérivés d'acide indolcarboxylique de formule générale

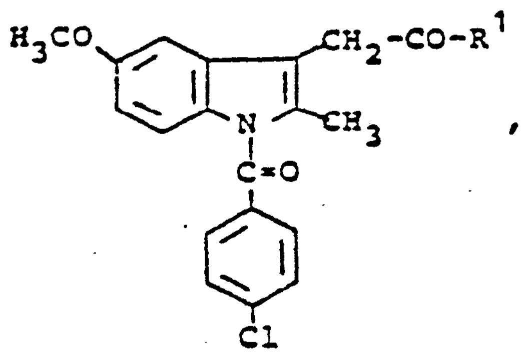

,

dans laquelle
$R^1$ représente les restes

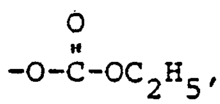, $-O-SO_2-CH_3$,

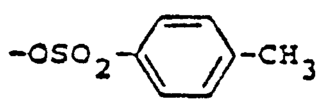

et

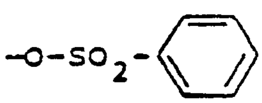

avec des composés de formule générale

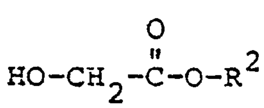

dans laquelle
$R^2$ représente l'hydrogène ou l'ion ammonium, en présence de solvants organiques inertes, dans un intervalle de température de −10°C à 80°C.

2. Procédé suivant la revendication 1, caractérisé en ce qu'on conduit la réaction dans l'intervalle de température de −10°C à +50°C.

3. Procédé suivant la revendication 1 ou 2, caractérisé en ce qu'on utilise comme solvants organiques inertes des éthers, des hydrocarbures chlorés, des amides substitués, des hydrocarbures aromatiques et/ou des cétones.

4. Procédé suivant la revendication 1 à 3, caractérisé en ce qu'on utilise comme dérivé d'acide indolcarboxylique un acide indolcarboxylique fonctionnalisé par formation d'anhydride avec le carbonate d'éthyle.

5. Procédé suivant les revendications 1 à 4, caractérisé en ce qu'on utilise un sel d'ammonium de l'acide glycolique.